# EUROPEAN PATENT SPECIFICATION

(11) **EP 1 765 848 B1**
(45) Date of publication and mention of the grant of the patent: **21.01.2009**
(21) Application number: 05759142.2
(22) Date of filing: 04.07.2005
(51) Int. Cl.: C07J 73/00

(54) **PROCESS FOR THE PREPARATION OF 4-AZASTEROIDS**
VERFAHREN ZUR HERSTELLUNG VON 4-AZASTEROIDEN
PROCÉDÉ POUR LA PRÉPARATION DE 4-AZASTÉROIDES

(30) Priority: 05.07.2004 EP 04015746
(43) Date of publication of application: 28.03.2007
(73) Proprietor: Lonza Group AG, 4052 Basel (CH)
(72) Inventor: BESSARD, Yves, CH-3960 Sierre (CH); KUO, David, Short Hills, NJ 07078 (US)
(86) International application number: PCT/EP2005/007190
(87) International publication number: WO 2006/002966

(56) References cited:
- WO-A-95/12398
- RASMUSSON G H ET AL: "AZASTEROIDS AS INHIBITORS OF RAT PROSTATIC 5ALPHA-REDUCTASE" JOURNAL OF MEDICINAL CHEMISTRY, AMERICAN CHEMICAL SOCIETY. WASHINGTON, US, vol. 27, no. 12, 1 December 1984 (1984-12-01), pages 1690-1701, XP002043194 ISSN: 0022-2623
- RASMUSSON G H ET AL: "AZASTEROIDS: STRUCTURE-ACTIVITY RELATIONSHIPS FOR INHIBITION OF 5ALPHA-REDUCTASE AND OF ANDROGEN RECEPTOR BINDING" JOURNAL OF MEDICINAL CHEMISTRY, AMERICAN CHEMICAL SOCIETY. WASHINGTON, US, vol. 29, no. 11, 1 November 1986 (1986-11-01), pages 2298-2315, XP000568779 ISSN: 0022-2623
- BRATOEFF E ET AL: "STEROIDAL ANTIANDROGENS AND 5ALPHA-REDUCTASE INHIBITORS" CURRENT MEDICINAL CHEMISTRY, BENTHAM SCIENCE PUBLISHERS BV, BE, vol. 6, no. 12, December 1999 (1999-12), pages 1107-1123, XP000874719 ISSN: 0929-8673
- ISHIBASHI, K. ET AL.: "Synthesis and testosterone 5 alpha-reductase inhibitory activity of 11-substituted 4-aza-5alpha-androstane compounds" EUROPEAN JOURNAL OF MEDICINAL CHEMISTRY, vol. 31, 1996, XP002306058

## Description

The invention relates to a process for the preparation of 4-azasteroids of the formula 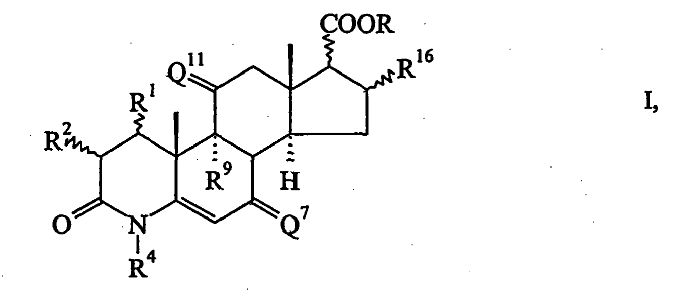 wherein R¹, R², R⁴, Q⁷, R⁹, Q¹¹, R¹⁶ and COOR are as defined below.

The three step process comprises (i) a haloform reaction of a 17-acetyl steroid converting the acetyl group into a -COOR group, (ii) subsequent ozonolysis of the A-ring and (iii) reclosure of the A-ring by reaction with an appropriate nitrogen compound of formula H₂NR⁴ to afford a compound of formula I.

Compounds of formula I are key intermediates for the production of pharmaceutically active 4-azasteroids, e.g. 17-substituted-4-aza-5α,17β-androsten-3-ones, among them Finasteride (Proscar^{®}, showing powerful 5α-reductase inhibition useful in the treatment of acne, alopecia and benign prostatic hypertrophy.

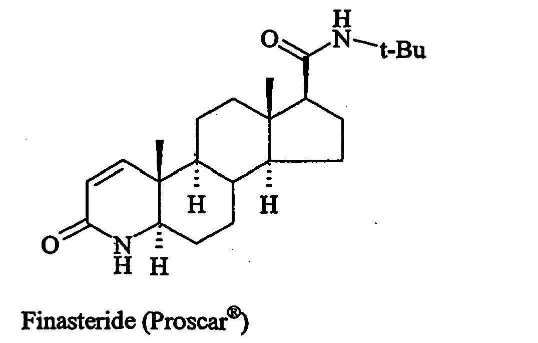

A detailed investigation on the inhibition activity of 5α-reductase by several 4-azasteroids and their preparation is disclosed in Rasmusson, Gary H. et al. J. Med. Chem. 29, 1986, 2298-2315.

Rasmusson, Gary H. et al. obtained 17β-carboxy-5-oxo-A-nor-3,5-secoandrostan-3-oic acid in about 75% yield by oxidation of 3-oxo-4-androstene-17β-carboxylic acid into using expensive oxidation agents such as NaIO₄ and KMnO₄.

WO 90/15045 provides a process wherein the A-ring and an unsaturated acyl side chain of a steroid comprising are ozonolyzed simultaneously. The disadvantage of WO 90/15045 is the removal of the unsaturated acyl side chain, which leads to a substantial material loss. In the examples 1 to 3 four carbon and three oxygen atoms or eight carbon atoms respectively are lost compared to the loss of one carbon atom in the instant process. Regarding the principles of "atom economy" introduced by Trost, B.M. (Science, 254,1991, 1471) a high loss of atoms in chemical reactions is disadvantageously.

EP-A-0 277 002 discloses in Scheme I the oxidation of several steroids with ruthenium dioxide/sodium periodate in an organic solvent leading to the corresponding acid. However, there is no example for said process and the Scheme contains no further details.

In saturated rigid ring structures like steroids, ring carbon atoms have
(a) either two monovalent substituents or one bivalent substituent attached to a carbon atom which is part of one ring and has two ring carbon-carbon bonds, or
(b) one monovalent substituent attached to a carbon atom which is part of two condensed rings and has three ring carbon-carbon bonds.

Each of the two substituents under (a) is in either an equatorial or axial position relative to the ring and may change between axial/equatorial due to ring flipping. However, the position of the two substituents relative to the ring and each other remain fixed. In structural formulae depicting such ring structures, accordingly to the common numbering of steroid systems, i.e. 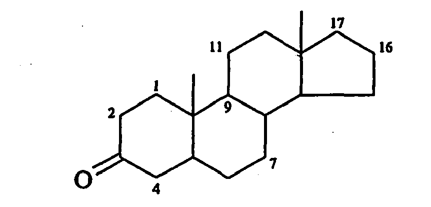 a substituent Rⁿ⁻¹, wherein n denotes the number of the respective carbon atom Cⁿ which is "below" the other substituent will be identified as being in the α-position (e.g. R¹⁻¹ or α-R¹) and its bond to the carbon atom Cⁿ is represented by a broken, dotted or dashed line. Accordingly, the substituent Rⁿ⁻² attached "above" the other (Rⁿ⁻¹) is identified as being in the β-position and its bond to the carbon atom Cⁿ is represented by a heavy or bold line or a solid wedge.

Herein, the symbol Qⁿ in structural formulae represents either one bivalent substituent or two monovalent substituents Rⁿ (i.e. Rⁿ⁻¹ or Rⁿ⁻²).

Where, according to (b), only one monovalent substituent is attached to the ring skeleton (e.g. R⁹ attached to C⁹), its relative position is described to be below (i.e. R⁹⁻¹) or above (i.e. R⁹⁻²) of the ring skeleton.

R¹ and R² are independently selected from the group consisting of hydrogen, F, Cl, Br, I, C₁₋₆-alkyl and C₁₋₆-alkoxy, wherein each alkyl and/or alkoxy moiety is optionally substituted with one or more halogen atoms.
R⁴ is selected from the group consisting of hydrogen, (*N,N-*di-C₁₋₆-alkylamino)methyl, 2-(*N,N-*di-C₁₋₆-alkylamino)ethyl, C₁₋₆-alkyl, C₁₋₆-alkoxy, phenyl and benzyl, wherein each alkyl and/or alkoxy moiety is optionally substituted with one or more halogen atoms, and wherein each phenyl and/or benzyl moiety is optionally substituted with one or more NO₂, F, Cl, Br, I, C₁₋₃-alkyl, C₁₋₃-alkoxy, dimethylamino or diethylamino groups.
Q⁷ represents a carbonyl oxygen atom or is R⁷⁻¹ and R⁷⁻², wherein one of R⁷⁻¹ and R⁷⁻² is hydrogen and the other is selected from the group consisting of hydrogen, F, Cl, Br, I, C₁₋₆-alkyl and C₁₋₆-alkoxy, wherein each alkyl and/or alkoxy moiety is optionally substituted with one or more halogen atoms.
R⁹ represents hydrogen or F.
Q¹¹ represents a carbonyl oxygen atom or is R¹¹⁻¹ and R¹¹⁻², wherein one of R¹¹⁻¹ and R¹¹⁻² is hydrogen and the other is selected from the group consisting of hydrogen, cyano, cyano-C₁₋₃-alkyl, acetoxy, COOH and COO⁻M⁺, wherein M⁺ is Na⁺, K⁺ or NH⁺₄.
R¹⁶ is selected from the group consisting of hydrogen, cyano, C₁₋₃-alkyl, cyano-C₁₋₃-alkyl, acetoxy, COOH and COO⁻M⁺, wherein M⁺ is Na⁺, K⁺ or NH⁺₄.
COOR represents COOH or COO⁻M⁺, wherein M⁺ is Na⁺, K⁺ or NH⁺₄.

Here and hereinbelow the term "C₁₋ₙ-alkyl" represents a linear or branched alkyl group having 1 to n carbon atoms. C₁₋₁₀-alkyl is for example methyl, ethyl, propyl, isopropyl, butyl, isobutyl, *sec*-butyl, *tert*-butyl, pentyl, 1,4-dimethyl-pentyl, hexyl, heptyl, 1,5-dimethyl-hexyl, decyl or 4-ethyl-1,5-dimethylhexyl.

Here and hereinbelow the term "C₁₋₆-alkoxy" represents a linear or branched alkoxy group having 1 to 6 carbon atoms, for example methoxy, ethoxy, propoxy, isopropoxy, butoxy, isobutoxy, sec-butoxy, pentyloxy or hexyloxy.

Here and hereinbelow the term "cyano-C₁₋₆-alkyl" represents a linear alkyl group having 1 to 6 carbon atoms with to a terminal cyano group, for example cyanomethyl, cyanoethyl, cyanopropyl, cyanobutyl, cyanopentyl or cyanohexyl.

Here and hereinbelow the term "C₃₋₁₈-alkanoic acid" represents a linear or branched alkanoic acid having 3 to 18 carbon atoms, for example propionic acid, butyric acid, octanoic acid, nonanoic acid, decanoic acid, undecanoic acid, dodecanoic acid or octadecanoic acid.

Here and hereinbelow the term "C₈₋₁₈-alkanesulfonic acid" represents a linear or branched alkanesulfonic acid having 8 to 18 carbon atoms, for example octanesulfonic acid, dodecanesulfonic acid.

Here and hereinbelow the term "C₁₋₆-alkylamino" represents a linear or branched alkylamino group having 1 to 6 carbon atoms, for example methylamino, ethylamino, propyl-amino, butylamino, pentylamino or hexylamino.

Here and hereinbelow the term "C₁₋₆-alkylamine" represents a linear or branched alkylamine having 1 to 6 carbon atoms, for example methylamine, ethylamine, propylamine, butylamine, pentylamine or hexylamine.

Here and hereinbelow the term "C₁₋₄-alcohol" represents a linear or branched alcohol having 1 to 4 carbon atoms, i.e. methanol, ethanol, propanol, isopropanol, *n*-butanol, sec-butanol and *tert*-butanol.

The technical problem to be solved was to provide an industrially applicable process for the preparation of steroidal seco acids wherein the oxidative cleavage of the steroidal A-ring can be separated from the formation of the carboxyl group attached to C¹⁷ of the steroidal skeleton. Furthermore the use of expensive agents like NaIO₄ should be avoided. A further object of the invention was to establish an economically advantageous reaction sequence for the preparation of 4-azasteroid intermediates of formula I, requiring a minimum of work-up and isolation steps for compounds of formula IV.

The problems above could be solved according to the process of claim 1.

To avoid the drawbacks in the known processes, a process was established which allows formation of the carboxyl group attached to C¹⁷ of the steroidal skeleton and purification of the compound of formula III prior to the cleavage of the steroidal A-ring. Since isolated oxidation of the ene-oxo side chain of the compounds disclosed in WO 90/15045 is not possible without infliction of the A-ring, the carboxyl group attached to C¹⁷ had to be introduced by a complete different reaction regime and different starting compounds. By subjecting to a haloform reaction compounds of formula II, containing an acetyl group attached to C¹⁷, can be converted to compounds of formula III easily and in high yield. The haloform reaction is completely indifferent to the ene-oxo fragment in the steroidal A-ring and compounds of formula III can be purified to comply with requirements for steroid drugs. The decoupling of both oxidation steps allows usage of starting compounds of poor quality.

Provided is a process for the preparation of 4-aza-steroidal acids of the formula 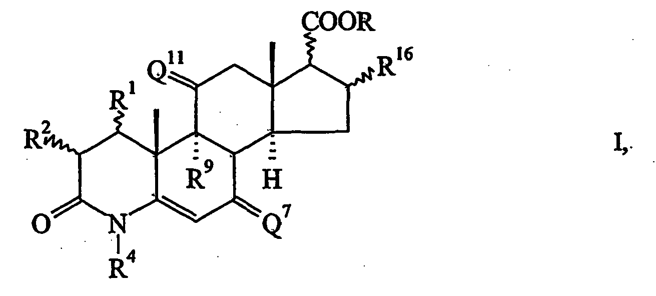 wherein
R¹, R², R⁴, Q⁷, R⁹, Q¹¹, R¹⁶ and COOR are as defined above,
comprising three steps of
(i) converting a compound of formula 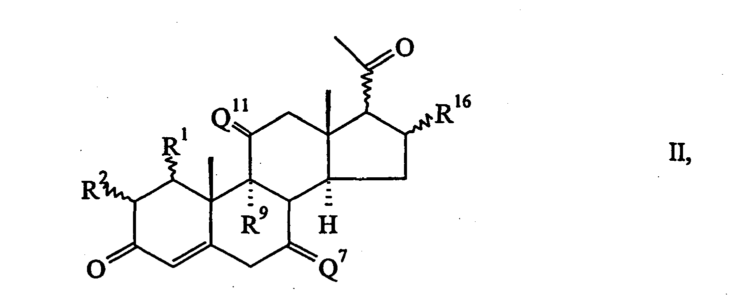 wherein R¹, R², Q⁷, R⁹, Q¹¹ and R¹⁶ are as defined above, by a haloform reaction, optionally in the presence of a polar organic additive, into a compound of formula 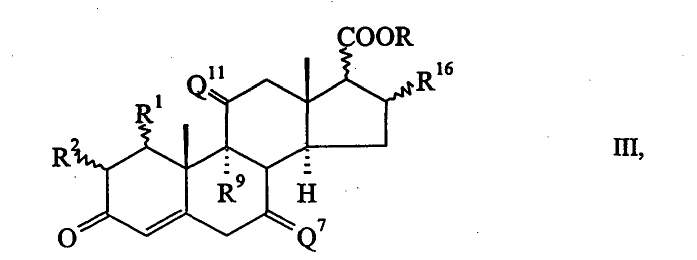 wherein R¹, R², Q⁷, R⁹, Q¹¹, R¹⁶ and COOR are as defined above, which is
(ii) reacted by ozonolysis and oxidative work-up procedure into a compound of formula 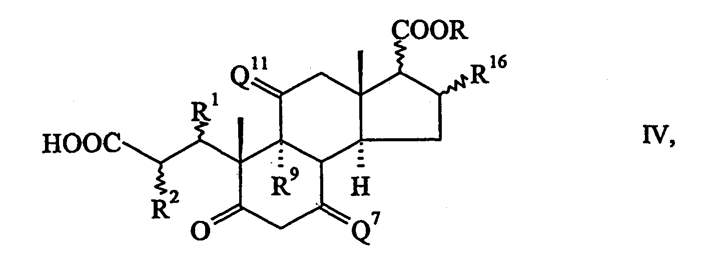 wherein R¹, R², Q⁷, R⁹, Q¹¹, R¹⁶ and COOR are as defined above, which is
(iii) cyclized with an nitrogen compound of the formula H₂NR⁴, wherein R⁴ is as defined above, into the compound of formula I.

In a preferred embodiment the haloform reaction of step (i) is a bromoform reaction.

Preferably said haloform reaction is carried out in aqueous solution, optionally in the presence of a polar organic additive. Such additive, being inert under the reaction conditions of the haloform reaction, may act as a solubilizer of the starting compound. In a preferred embodiment the polar organic additive is selected from the group consisting of *tert*-butylalcohol, acetonitrile and propionitrile.

Preferably step (i) is carried out in the presence of a hydroxide ion releasing base. Such a base can be an alkali and/or earth alkali hydroxide or a bicarbonate or carbonate, which is able to release hydroxide ions by deprotonating water present in the reaction mixture. Particularly preferred the base is selected from the group consisting of LiOH, NaOH, KOH, Ba(OH)₂, Mg(OH)₂, Ca(OH)₂, NaHCO₃, KHCO₃, Na₂CO₃, K₂CO₃ and mixtures thereof, more preferably NaOH, KOH, NaHCO₃ and mixtures thereof.

Optionally, step (i) is followed by a purification step. Due to the higher polarity and the possibility of form salt formation of compounds of formula III may be separated from compounds of formula II by liquid-liquid extraction. Another appropriate purification method is recrystallization of the product in a C₁₋₄-alcohol/water mixture. Preferably a methanol/water mixture at a temperature of 30 to 80 °C is applied.

Advantageously the ozonolysis is carried out by passing ozone through a solution of a compound of formula III in an inert solvent. Preferably the solvent of step (ii) is selected from the group consisting of H₂O, C₁₋₄-alcohols, acetonitrile, propionitrile, methylene chloride and chloroform.

In a preferred embodiment step (ii) is carried out at a temperature below -10 °C, more preferably at or below -15 °C.

The ozonolysis is applied until a sufficient conversion is reached, then oxygen is passed through the reaction mixture to remove at least the major amount of excess ozone. For the oxidative work-up procedure a hydroxide ion releasing base is added to the reaction mixture. A suitable hydroxide ion releasing base is alkali and/or earth alkali hydroxide or a bicarbonate or carbonate, which is able to release hydroxide ions by deprotonating water present in the reaction mixture. Particularly preferred the base is selected from the group consisting of LiOH, NaOH, KOH, Ba(OH)₂, Mg(OH)₂, CaOH)₂, NaHCO₃, KHCO₃, Na₂CO₃, K₂CO₃ and mixtures thereof, more preferably NaOH, KOH, NaHCO₃ and mixtures thereof. Completion of the oxidative work-up procedure can be followed for example by redox titration or polarography.

When the oxidative work-up procedure is finished for removal of residual ozone and possibly formed peroxides, the reaction mixture is then treated with a reducing agent In a preferred embodiment the reducing agent is selected from the group consisting of NaHSO₃, KHSO₃, SO₂, tri-C₁₋₃-alkyl-phosphines, tri-C₁₋₃-alkyl-phosphites, triphenylphosphine and mixtures thereof, preferably NaHSO₃ or SO₂.

Particularly preferred the reaction mixture is maintained at a temperature between 20 and 100 °C for at least 5 minutes after addition of the reducing agent Complete removal of peroxides and ozone is time and temperature dependent At 60 °C a reduction time of below 1 hour is often sufficient, at 100 °C some minutes will be sufficient

After the ozonolyis and oxidative work-up procedure has finished the salts of the acids of formula III remain in the basic aqueous solution. To obtain the protonated compounds of formula III preferably a liquid-liquid extraction is applied. More preferably before the extraction the pH of the aqueous phase is adjusted to below pH 7, more preferably between pH 3 and 6.

In another particularly preferred embodiment after removal of excess oxidants and adjusting the pH to below 7, Celite^{®} is added to the aqueous solution and the mixture is filtered to remove sticky compounds and to enhance the product quality.

In a further particularly preferred embodiment the liquid-liquid extraction is carried out using tert-butyl methyl ether (TBME), CHCl₃, CH₂Cl₂, 1,1,1-trichloroethane, 1,2-dichloropropane, methyl acetate or ethyl acetate. Preferably the extraction of the aqueous mixture is carried out with ethyl acetate at an pH between 4.5 and 5.5.

In a preferred process using CH₂Cl₂ for the extraction, optionally the product is precipitated from CH₂Cl₂ by change of the polarity of the solvent, by adding an apolar solvent like methyl cyclohexane.

Preferably, the cyclization reaction of step (iii) is carried out between 80 and 150 °C, particularly preferred between 110 and 140 °C.

In a further preferred embodiment, the cyclization reaction is carried out in the presence of a non-oxidizing proton acid and/or a polar organic additive to facilitate solvatisation. Preferably the non-oxidizing proton acid is an organic or inorganic acid selected from the group consisting of acetic acid, propionic acid, butyric acid, isobutyric acid, benzoic acid, HCl, HBr, HI and mixtures thereof. Particularly preferred, the proton acid is acetic or propionic acid.

Preferably the polar organic additive is selected from the group consisting of *tert*-butyl methyl ether, C₁₋₄-alkyl alcohols, ethylene glycol, acetonitrile and dimethyl sulfoxide.

In a preferred embodiment R⁴ in the nitrogen compound of formula H₂NR⁴ is hydrogen or C₁₋₆-alkyl, optionally substituted with one or more halogen atoms. In a further preferred embodiment soluble salts of the nitrogen compounds of formula H₂NR⁴ are used, for example NH₄Cl, NH₄Br, ammonium acetate, ammonium propionate, ammonium butyrate, or hydrochlorides or hydrobromides of C₁₋₆-alkylamines. In a further preferred embodiment the nitrogen compound of the formula H₂NR⁴ or a salt thereof can be used as a solution in the presence of a non-oxidizing proton acid and/or a polar additive, as defined above.
Particularly preferred the nitrogen compound is selected from the group consisting of C₁₋₆-alkylamines, soluble salts thereof, NH₃, NH₄Cl NH₄Br and NH₄OAc, preferably NH₃ and NH₄OAc.

In a particularly preferred embodiment 3-oxo-4-aza-androst-5-ene-17β-carboxylic acid of formula 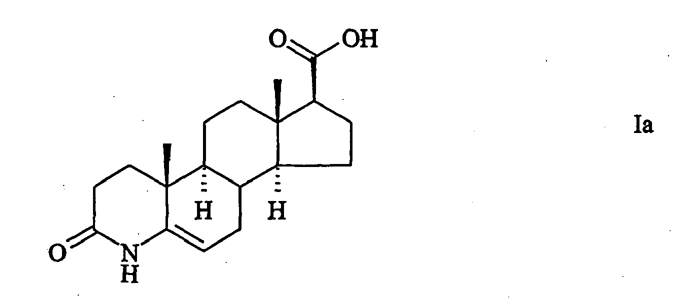 is prepared, wherein
(i) progesterone of formula 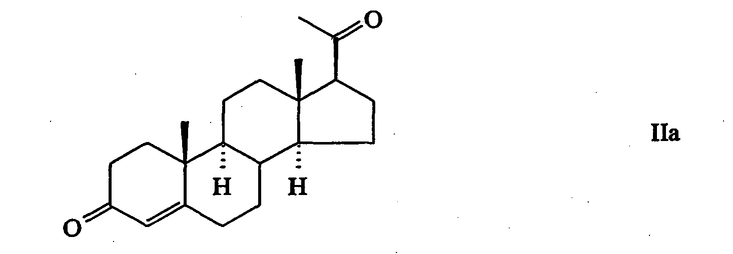 or a salt thereof, is reacted by a bromoform reaction, optionally in the presence of a polar organic additive, into progesteronic acid of formula 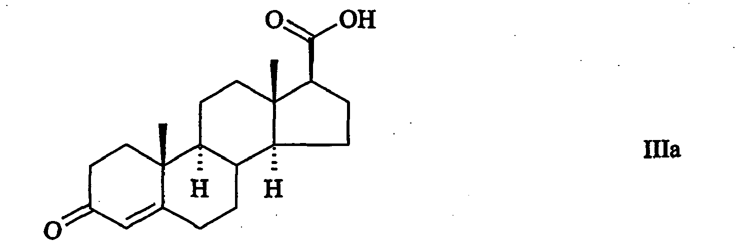 or a salt thereof, which is,
(ii) reacted by ozonolysis and oxidative work-up into 17β-carboxy-5-oxo-A-nor-3,5-secoandrostan-3-oic acid of formula 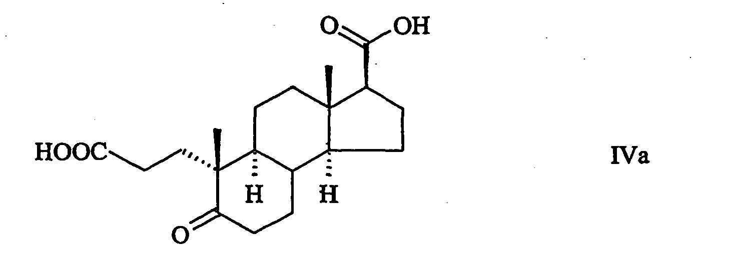 or a salt thereof, which is
(iii) cyclized by reaction with NH₃ or a soluble salt thereof, preferably with NH₃ in ethylene glycol or NH₄OAc in AcOH, into compound Ia or a salt thereof.

In a preferred embodiment the bromoform reaction of compound IIa in step (i) is carried out in the presence of *tert*-butanol.
The ozonolysis of compound IIIa in step (ii) is preferably carried out in methanol below - 10 °C. In a further preferred embodiment the oxidative work-up is carried out at a pH >9. After oxidative work-up is finished, peroxides are preferably removed using NaHSO₃ as reducing agent.
In a preferred embodiment cyclization of compound IVa in step (iii) is carried out with NH₄OAc at a temperature of 110 to 140 °C. Optionally step (iii) is carried out in the presence of a non-oxidizing proton acid, preferably in the presence of acetic acid.

The present invention is illustrated by the following non-limiting examples.

### Examples:

### Example 1: 3-Oxo-4-androst-4-ene-17β-carboxylic acid (IIIa of formula III with R¹ = R² = R⁷⁻¹= R⁷⁻² = R⁹ = R¹¹⁻¹ = R¹¹⁻² = R¹⁶ = H, COOR = COOH)

A three-neck 1 L reactor equipped with a mechanical stirrer and thermometer is charged, under nitrogen atmosphere, with 30% NaOH (379 g, 2840 mmol, 9.46 eq) and distilled water (459 mL). The NaOH the solution was cooled to 0 to 5 °C, and bromine (214.8 g, 1344 mmol; 4.48 eq) was introduced over a period of at least 60 min to form an orange solution of sodium hypobromite. The solution was then stirred an additional 30 min at 0 °C prior to use. A three-neck 3 L reactor equipped with a mechanical stirrer, thermometer and distillation head is charged, under nitrogen atmosphere, with progesterone (IIa, 94.5g, 300 mmol, 1.0 eq), *tert*-butanol (910 mL) and stirred at 25 °C. A-solution of 30% NaOH (197 g, 1477 mmol, 4.92 eq) and distilled water (474 mL) was then added over a period of 70 min, while the temperature was held at 25 °C. The yellow solution was cooled to 0 °C before being treated with NaOBr solution over a period of 60 min. The heterogeneous mixture was then stirred for 3 h at 0 °C.

After completion of the reaction a solution of sodium sulfite (37,8 g, 364 mmol, 1.21 eq) in water (147 mL) was added within 15 min to the cold reaction mixture (slightly exothermic). The solution was aged overnight (15 h) at 20 °C. *tert*-Butylalcohol was then removed by distillation at 23 to 37 °C and a pressure of 90 to 65 mbar. 807 g of alcohol were removed from the reaction mixture in this way. Distilled water (1200 mL) was then added to the rose coloured reaction mixture, followed by toluene (300 mL), and the mixture warmed to 35 °C. After 15 min the stirring was stopped and the phases allowed to separate. The separated aqueous phase (2780 g, pH 14) was then acidified to pH 3 over a 90 minute period by the addition of 37% HCl (244 g, 2476 mmol, 8.25 eq), and the resulting solution was stirred at room temperature for an additional 60 min before being filtered. The filter cake was washed with distilled water (2×180 mL) and the wet product (217 g) dried under vacuum (45 °C, approx. 20 mbar). 85.5 g of IIIa (69%, HPLC 77% w/w, 94% area) of 3-oxo-4-androst-4-ene-17β-carboxylic acid (etiocholenic acid) was isolated.

### Example 2: Purification of 3-oxo-4-androst-4-ene-17β-carboxylic acid (IIIa of formula III with R¹ = R² = R⁷⁻¹ = R⁷⁻² = R⁹ = R¹¹⁻¹ = R¹¹⁻² = R¹⁶ = H, COOR = COOH)

A three-neck 2 L reactor equipped with a mechanical stirrer, thermometer and distillation head is charged, under nitrogen atmosphere, with crude etiocholenic acid (170 g, HPLC 77% w/w), methanol (1360 mL), and distilled water (340 mL) stirred and then warmed to reflux (65 °C). The suspension was stirred for 2 h. and then cooled to 0 °C over a 75 min period. The resulting suspension was filtrated and the filter cake was washed with cooled (60:40) methanol / water solution (2×100 mL). After drying under vacuum (45 °C, approx. 20 mbar), 110 g (84%, HPLC 99% w/w, 99% area) of pure etiocholenic acid-(IIIa) were obtained.

### Example 3: 17β-Carboxy-5-oxo-A-nor-3,5-secoandrostan-3-oic acid (IVa of formula IV with R¹ = R² = R⁷⁻¹ = R⁷⁻² = R⁹ = R¹¹⁻¹ = R¹¹⁻² = R¹⁶ = H, COOR = COOH)

Etiocholenic acid (IIIa, 79.1 g, 250 mmol) was placed in the ozonolysis vessel under nitrogen and taken up in methanol (750 mL). The mixture was cooled to -15 °C (internal temperature), and treated with ozone for about 4 hours after reaction were completed the flow of ozone was stopped and O₂ was bubbled through the mixture for 15 min. The reaction mixture was then placed under N₂, and water (425 mL) was then added at the same temperature, resulting the precipitation of a white solid. Over the following 2 h the mixture was allowed to warm from -15 to 40 °C. For oxidative work-up, the reaction mixture is taken up to pH 11 by addition of 30% sodium hydroxide (93 mL). The reaction mixture was concentrated under vacuum (40 °C, approx. 150 mbar) (492 g, distillate) and the remaining yellow solution (713 g) extracted, at room temperature, with *tert-*butyl methyl ether (500 mL). The organic phase was separated and treated with 40% NaHSO₃ (1×25 mL) to eliminate peroxides, then concentrated on the rotorvapor (35 °C and 30 mbar) to give a light yellow product (100 g). The residual aqueous solution was then over a period of 30 min adjusted to pH 3 by treating with 35% HCl (65 mL) at room temperature, before being extracted with *tert-*butyl methyl ether (600 mL). Separation of the organic solution, followed by washing with 40% NaHSO₃ (1 × 25 mL) to eliminate peroxides and then with water (1 × 25 mL). By concentration on a rotorvapor, (35 °C, 20 mbar) and drying (16 h) under vacuum 92.7 g (HPLC 72% w/w, 73% area) fine white powder of 17β-carboxy-5-oxo-A-nor-3,5-secoandrostan-3-oic acid (IVa) were obtained.

### Example 4: 3-Oxo-4-aza-androst-5-ene-17β-carboxylic acid (Ia of formula I with R¹ = R² = R⁴ = R⁷⁻¹ = R⁷⁻² = R⁹ = R¹¹⁻¹ = R¹¹⁻² = R¹⁶ = H, COOR = COOH)

A three-neck 1000 mL reactor equipped with a mechanical stirrer, a thermometer and a distillation head is charged, under nitrogen atmosphere, with a *tert-*butyl methyl ether solution of 17β-carboxy-5-oxo-A-nor-3,5-secoandrostan-3-oic acid (IVa, 700 mL). The solvent is distilled under vacuum (100 mbar / bath approx. 60 °C) to 1/3 of the initial volume. Acetic acid (760 mL) is then added and distillation continued until acetic acid starts to distil. Ammonium acetate (132.5 g, 1700 mmol, approx. 7 eq) is added and the reaction mixture is heated to reflux (approx. 120 °C) within 1 h (bath temp.: approx. 140 °C). After 1 h and 2 h, the conversion is checked by TLC. The reaction mixture is then concentrated (40% of acetic acid distilled off). The reaction mixture (yellow-orange suspension) is cooled down within 1 h to approx. 60 °C. Water (830 mL) is then slowly added (approx. 1 h) to the reaction mixture at approx. 60 °C and the suspension slowly (1 h) cooled to 20 °C. After 0.5 h (20 °C), the suspension is filtered and the filter cake was washed with water (2×100 mL) and a solution of water/ethanol (1:1; v:v) (2×100 mL). Drying at 80 °C (approx. 20 mbar / approx. 16 h) afforded 68.9g (83% overall yield, assay corrected, from etiocholenic acid) of light tan product of 3-oxo-4-aza-androst-5-ene-17β-carboxylic acid (Ia)-(HPLC 99% area, 95.4% w/w).

### Example 5: 3-Oxo-4-aza-androst-5-ene-17β-carboxylic acid (Ia of formula I with R¹= R² = R⁴ = R⁷⁻¹ = R⁷⁻² = R⁹ = R¹⁻¹ = R¹⁻² = R¹⁶ = H, COOR = COOH)

To the conc. solution of 17β-carboxy-5-oxo-A-nor-3,5-secoandrostan-3-oic acid (IVa) (450 g, 1.33 mol), 2100 mL of acetic acid are added and ethyl acetate is distilled off. The cooled mixture is then transferred, under nitrogen atmosphere, to a three-neck 10 L reactor, equipped with a mechanical stirrer, a thermometer and a distillation head, and than charged with ammonium acetate (560 g, 7.2 mol). The reaction mixture is heated to reflux within 1 h and than cooled down within 0.5 h to 60 °C. Water (3360 mL) is then slowly added and the suspension slowly cooled to 20°C. After 0.5 h, the suspension is filtered and the filter cake was washed with water (2x840 mL) and a mixture of water/ethanol [v:v, 1:1] (3x840 mL). Drying at 50°C (approx. 20 mbar / approx. 16 h) affords 355 g (74.5% overall yield) of white to light tan of 3-oxo-4-aza-androst-5-ene-17β-carboxylic acid-(Ia) at a purity of about 100%.

### Comparative example 1: 17β-Carboxy-5-oxo-A-nor-3,5-secoandrostan-3-oic acid (Formula I, R¹ = R² = R⁷⁻¹ = R⁷⁻² = R⁹ = R¹¹⁻¹ = R¹¹⁻² = R¹⁶ =H, COOR = COOH)

A three-neck 1000 mL reactor, equipped with a mechanical stirrer, a thermometer, a device for pH-controlled NaOH addition and a bubble counter, is purged with nitrogen and then charged with 200 mL of deionized water, 9.7 g of NaHCO₃ (115.5 mmol), 16.2 g of etiocholenic acid (50 mmol) and 58 mg of RuCl₃·H₂O (0.37 mmol) and 16 mL of acetonitrile. The slurry is cooled down to 10 °C under vigorous stirring (approx. 1000 rpm). The solution is first adjusted to pH 8.0 by addition of NaOH (30%). A solution of 105.9 g of OXONE^{®} (345 mmol, monopersulfate compound 2KHSO₅·KHSO₄·K₂SO₄, from Aldrich) in 425 mL of deionized water is added to the stirred suspension within 3.5 h, while the mixture is maintained between pH 8.0 and 8.4 by addition of 30% NaOH solution. The reaction mixture is aged for 1.5 h. After complete conversion of etiocholenic acid, 4.3 g of NaHSO₃ (40 mmol) is added. The reaction mixture is warmed up to 60 °C for 1 h (colour changes from brown to light green) and then cooled to 20°C. CH₂Cl₂ (254 mL) is added. The pH is adjusted to 1.5 by the addition of conc. HCl. Alter phase separation the water phase is extracted with 80 mL CH₂Cl₂. The combined organic layers are then filtered, dried over Na₂SO₄. Residual water is removed from the moist product at 55 °C under vacuum. Yield is approx. 90%, by HPLC, purity is approx. 60%.

## Claims

1. A process for the preparation of 4-azasteroids of the formula 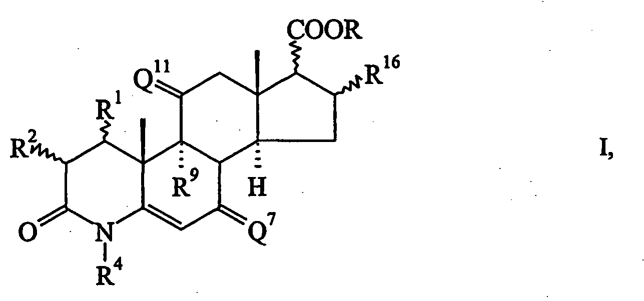 wherein
R¹ and R² are independently selected from the group consisting of hydrogen, F, Cl, Br, I, C₁₋₆-alkyl and C₁₋₆-alkoxy, wherein each alkyl and/or alkoxy moiety is optionally substituted with one or more halogen atoms, and
R⁴ is selected from the group consisting of hydrogen, (*N,N*-di-C₁₋₆-alkylamino)-methyl, 2-(*N,N*-di-C₁₋₆-alkylamino)ethyl, C₁₋₆-alkyl, C₁₋₆-alkoxy, phenyl and benzyl, wherein each alkyl and/or alkoxy moiety is optionally substituted with one or more halogen atoms, and wherein each phenyl and/or benzyl moiety is optionally substituted with one or more NO₂, F, Cl, Br, I, C₁₋₃-alkyl, C₁₋₃-alkoxy, dimethylamino or diethylamino groups, and
Q⁷ represents a carbonyl oxygen atom or is R⁷⁻¹ and R⁷⁻², wherein one of R⁷⁻¹ and
R⁷⁻² is hydrogen and the other is selected from the group consisting of hydrogen, F, Cl, Br, I, C₁₋₆-alkyl and C₁₋₆-alkoxy, wherein each alkyl and/or alkoxy moiety is optionally substituted with one or more halogen atoms, and
R⁹ represents hydrogen or F, and
Q¹¹ represents a carbonyl oxygen atom or is R¹¹⁻¹ and R¹¹⁻², wherein one of R¹¹⁻¹ and
R¹¹⁻² is hydrogen and the other is selected from the group consisting of hydrogen, cyano, cyano-C₁₋₃-alkyl, acetoxy, COOH and COO⁻M⁺, wherein M⁺ is Na⁺, K⁺ or NH⁺₄, and
R¹⁶ is selected from the group consisting of hydrogen, cyano, C₁₋₃-alkyl, cyano-C₁₋₃-alkyl, acetoxy, COOH and COO⁻M⁺, wherein M⁺ is Na⁺, K⁺ or NH⁺₄, and COOR is COOH or COO⁻M⁺, wherein M⁺ is Na⁺, K⁺ or NH⁺₄,
comprising three steps of
(i) converting a compound of formula 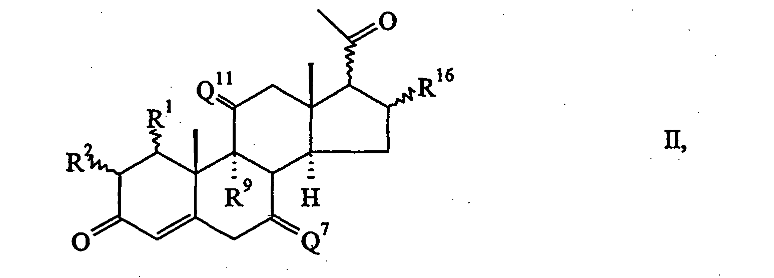 wherein R¹, R², Q⁷, R⁹, Q¹¹, and R¹⁶ are as defined above, by a haloform reaction, optionally in the presence of a polar organic additive, into a compound of formula 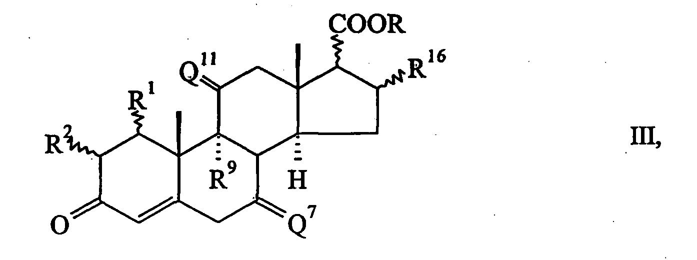 wherein R¹, R², Q⁷, R⁹, Q¹¹, R¹⁶ and COOR are as defined above, which is
(ii) reacted by ozonolysis and oxidative work-up procedure into a compound of formula 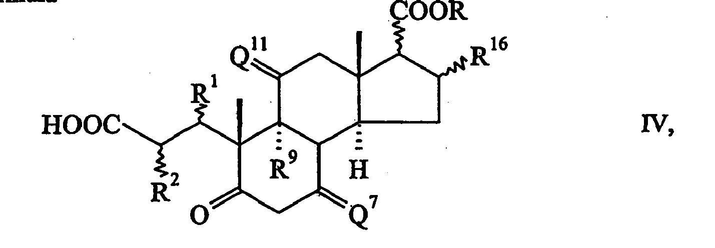 wherein R¹, R², Q⁷, R⁹, Q¹¹, R¹⁶ and COOR are as defined above, which is
(iii) cyclized with a nitrogen compound of the formula H₂NR⁴, wherein R⁴ is as defined above, into the compound of formula I, wherein R¹, R², R⁴, Q⁷ R⁹, Q¹¹, R¹⁶ and COOR are as defined above.

2. The process of claim 1, wherein the haloform reaction is a bromoform reaction.

3. The process of claims 1 or 2, wherein the polar organic additive is selected from the group consisting of *tert*-butylalcohol, acetonitrile and propionitrile.

4. The process of any of claims 1 to 3, wherein step (ii) is carried out in a solvent selected from the group consisting H₂O, C₁₋₄-alcohol, acetonitrile, propionitrile, methylene chloride and chloroform.

5. The process of any of claims 1 to 4, wherein at the end of step (ii) after oxidative work-up procedure a reducing agent is added to the reaction mixture to remove peroxides.

6. The process of any of claims 1 to 5, wherein in the nitrogen compound of the formula H₂NR⁴ in step (iii) is selected from the group consisting of C₁₋₆-alkylamines, soluble salts thereof, NH₃, NE₄Cl, NH₄Br and NH₄OAc.

7. The process of any of claims 1 to 6, wherein step (iii) is carried out at a temperature of 80 to 150 °C, preferably of 110 to 140 °C.

8. The process of claims 1 to 7, wherein step (iii) is carried out in the presence of a non-oxidizing proton acid selected from the group consisting of acetic acid, propionic acid, butyric acid, isobutyric acid, benzoic acid, HCl, HBr and HI.

9. The process of any of claims 1 to 8, wherein step (iii) is carried out in the presence of a polar organic additive selected from the group consisting of tert-butyl methyl ether, C₁₋₄-alkyl alcohols, ethylene glycol, acetonitrile and dimethyl sulfoxide.

10. The process of any of claims 1 to 9, for the preparation of 3-oxo-4-aza-androst-5-ene-17β-carboxylic acid of formula I with R¹ = R² = R⁴ = R⁷⁻¹= R⁷⁻² = R⁹ = R¹¹⁻¹ = R¹¹⁻² = R¹⁶ = H and COOR = COOH (Ia) wherein step (ii) is a bromoform reaction, and wherein step (iii) is carried out by reacting 17β-carboxy-5-oxo-A-nor-3,5-secoandrostan-3-oic acid of formula IVa or a salt thereof with NH₃ or a soluble ammonium salt, preferably with NH₃ in ethylene glycol or NH₄OAc in AcOH, into compound Ia or a salt thereof.

## Patentansprüche

1. Ein Verfahren zur Herstellung von 4-Azasteroiden der Formel 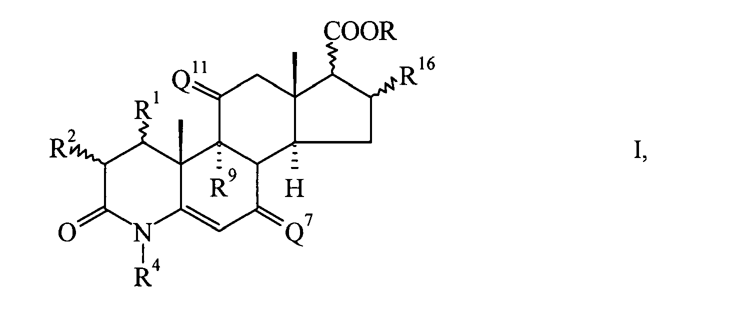 worin
R¹ und R² unabhängig voneinander ausgewählt sind aus der Gruppe bestehend aus Wasserstoff, F, Cl, Br, I, C₁₋₆-Alkyl und C₁₋₆-Alkoxy, worin jede Alkyl- und/oder Alkoxygruppe gegebenenfalls mit einem oder mehreren Halogenatomen substituiert ist, und
R⁴ ausgewählt ist aus der Gruppe bestehend aus Wasserstoff, (*N,N*-Di-C₁₋₆-alkyl-amino)methyl, 2-(*N,N*-Di-C₁₋₆-alkylamino)ethyl, C₁₋₆-Alkyl, C₁₋₆-Alkoxy, Phenyl und Benzyl, worin jede Alkyl- und/oder Alkoxygruppe gegebenenfalls mit einem oder mehreren Halogenatomen substituiert ist, und worin jede Phenyl- und/oder Benzylgruppe gegebenenfalls mit einem oder mehreren Halogenatomen NO₂, F, Cl, Br, I, C₁₋₃-Alkyl, C₁₋₃-Alkoxy, Dimethylamino or Diethylaminogruppen substituiert ist, und
Q⁷ repräsentiert ein Carbonylsauerstoffatom oder ist R⁷⁻¹ und R⁷⁻², worin eine der Gruppen R⁷⁻¹ oder R⁷⁻² Wasserstoff ist, und das andere ausgewählt ist aus der Gruppe bestehend aus Wasserstoff, F, Cl, Br, I, C₁₋₆-Alkyl und C₁₋₆-Alkoxy, worin jede Alkyl- und/oder Alkoxygruppe gegebenenfalls mit einem oder mehreren Halogenatomen substituiert ist, und
R⁹ repräsentiert Wasserstoff oder F, und
Q¹¹ repräsentiert ein Carbonylsauerstoffatom oder ist R¹¹⁻¹ und R¹¹⁻², worin eine der Gruppen R¹¹⁻¹ oder R¹¹⁻² Wasserstoff ist, und die andere ausgewählt ist aus der Gruppe bestehend aus Wasserstoff, Cyano, Cyano-C₁₋₃-alkyl, Acetoxy, COOH und COO⁻M⁺, worin M⁺ Na⁺, K⁺ oder NH⁺₄ ist, und
R¹⁶ ist ausgewählt aus der Gruppe bestehend aus Wasserstoff, Cyano, Cyano-C₁₋₃-alkyl, Acetoxy, COOH und COO⁻M⁺, worin M⁺ Na⁺, K⁺ oder NH⁺₄ ist, und COOR ist COOH oder COO⁻M⁺, worin M⁺ Na⁺, K⁺ oder NH⁺₄ ist, umfassend die drei Schritte
(i) Umwandeln einer Verbindung der Formel 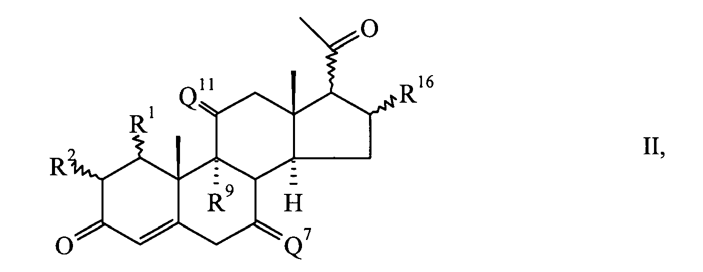 worin R¹, R², Q⁷, R⁹, Q¹¹ und R¹⁶ wie oben definiert sind, durch eine Haloformreaktion, gegebenenfalls in der Gegenwart eines polaren organischen Zusatzes, um eine Verbindung der Formel 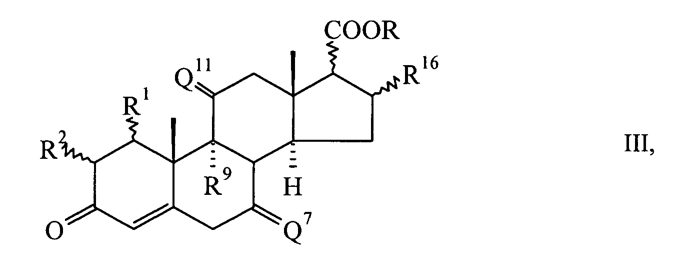 worin R¹, R², Q⁷, R⁹, Q¹¹, R¹⁶ und COOR wie oben definiert sind, welches
(ii) durch Ozonolyse und oxidative Aufarbeitung umgesetzt wird zu einer Verbindung der Formel 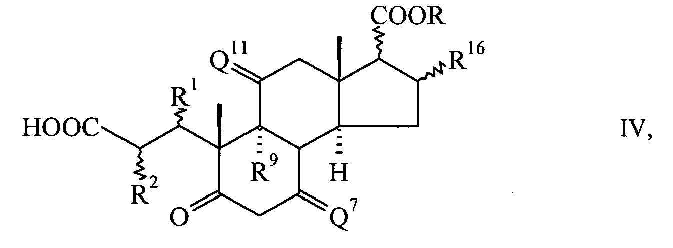 worin R¹, R², Q⁷, R⁹, Q¹¹, R¹⁶ und COOR wie oben definiert sind, welches
(iii) mit einer Stickstoffverbindung der Formel H₂NR⁴, worin R⁴ wie oben definiert ist, zur Verbindung der Formel I cylisiert wird, worin R¹, R², R⁴, Q⁷, R⁹, Q¹¹, R¹⁶ und COOR wie oben definiert sind.

2. Verfahren gemäss Anspruch 1, worin die Haloformreaktion eine Bromoformreaktion ist.

3. Verfahren gemäss Anspruch 1 oder 2, worin das polare organische Additiv ausgewählt ist aus der Gruppe bestehend aus tert-Butylalkohol, Acetonitril und Propionitril.

4. Verfahren gemäss einem der Ansprüche 1 bis 3, worin Schritt (ii) wird in einem Lösungsmittel durchgeführt, ausgewählt aus der Gruppe bestehend H₂O, C₁₋₄-Alkohol, Acetonitril, Propionitril, Methylenchlorid und Chloroform.

5. Verfahren gemäss einem der Ansprüche 1 bis 4, worin, nach dem oxidativen Aufarbeitungsschritt am Ende von Schritt (ii), der Reaktionsmischung ein Reduktionsmittel zugefügt wird um Peroxide zu entfernen.

6. Verfahren gemäss einem der Ansprüche 1 bis 5, worin die Stickstoffverbindung der Formel H₂NR⁴ in Schritt (iii) ausgewählt ist aus der Gruppe bestehend aus C₁₋₆-Alkylaminen und löslichen Salzen davon, NH₃, NH₄Cl, NH₄Br und NH₄OAc.

7. Verfahren gemäss einem der Ansprüche 1 bis 6, worin Schritt (iii) bei einer Temperatur von 80 bis 150 °C ausgeführt wird, bevorzugt bei 110 bis 140 °C.

8. Verfahren gemäss einem der Ansprüche 1 bis 7, worin Schritt (iii) in Gegenwart einer nichtoxidierenden Protonensäure ausgewählt aus der Gruppe bestehend aus Essigsäure, Propionsäure, Buttersäure, Isobuttersäure, Benzoesäure, HCl, HBr und HI.

9. Verfahren gemäss einem der Ansprüche 1 bis 8, worin Schritt (iii) in Gegenwart eines polaren organische Additivs, ausgewählt aus der Gruppe tert-Butylmethylether, C₁₋₄-Alkylalkohole, Ethylenglycol, Acetonitril und Dimethylsulfoxid.

10. Verfahren gemäss einem der Ansprüche 1 bis 9, für die Herstellung einer 3-Oxo-4-aza-androst-5-en-17β-carbonsäure der Formel I mit R¹ = R² = R⁴ = R⁷⁻¹ = R⁷⁻² = R⁹ = R¹¹⁻¹ = R¹¹⁻² = R¹⁶ = H und COOR = COOH (Ia), worin Schritt (ii) eine Bromoform-reaktion ist, und worin Schritt (iii) ausgeführt wird durch Reaktion einer 17β-Carboxy-5-oxo-A-nor-3,5-secoandrostan-3-on säure der Formel IVa oder einem Salz davon mit NH₃ oder einem löslichen Ammoniumsalz, vorzugsweise mit NH₃ in Ethylenglycol oder NH₄OAc in AcOH, zu einer Verbindung Ia oder einem Salz davon.

## Revendications

1. Procédé de préparation de 4-azastéroïdes de formule 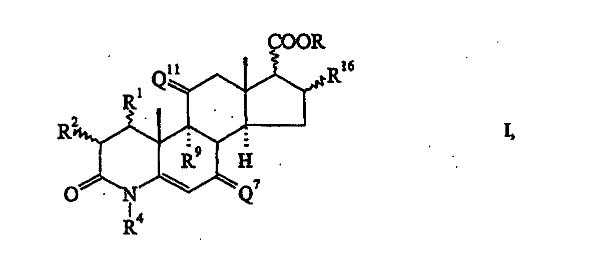 dans laquelle
R¹ et R² sont choisis indépendamment parmi le groupe constitué d'un atome d'hydrogène, d'un atome de fluor, d'un atome de chlore, d'un atome de brome, d'un atome d'iode, d'un groupe alkyle en C₁₋₆ et d'un groupe alcoxy en C₁₋₆, où chaque fragment alkyle et/ou alcoxy est éventuellement substitué par un ou plusieurs atomes d'halogène, et
R⁴ est choisi parmi le groupe constitué d'un atome d'hydrogène, d'un groupe (*N,N-*di-C₁₋₆-alkylamino) méthyle, d'un groupe 2- (*N,N*-di-C₁₋₆-alkylamino) éthyle, d'un groupe alkyle en C₁₋₆, d'un groupe alcoxy en C₁₋₆, d'un groupe phényle et d'un groupe benzyle, où chaque fragment alkyle et/ou alcoxy est éventuellement substitué par un ou plusieurs atomes d'halogène, et où chaque fragment phényle et/ou benzyle est éventuellement substitué par un ou plusieurs groupes NO₂, atomes de fluor, atomes de chlore, atomes de brome, atomes d'iode, groupes alkyle en C₁₋₃, groupes alcoxy en C₁₋₃, groupes diméthylamino ou groupes diéthylamino, et
Q⁷ représente un atome d'oxygène de carbonyle ou est R⁷⁻¹ et R⁷⁻², où l'un parmi R⁷⁻¹ et R⁷⁻² est un atome d'hydrogène et l'autre est choisi parmi le groupe constitué d'un atome d'hydrogène, d'un atome de fluor, d'un atome de chlore, d'un atome de brome, d'un atome d'iode, d'un groupe alkyle en C₁₋₆ et d'un groupe alcoxy en C₁₋₆, où chaque fragment alkyle et/ou alcoxy est éventuellement substitué par un ou plusieurs atomes d'halogène, et
R⁹ représente un atome d'hydrogène ou un atome de fluor, et
Q¹¹ représente un atome d'oxygène de carbonyle ou est R¹¹⁻¹ et R¹¹⁻², où l'un parmi R¹¹⁻¹ et R¹¹⁻² est un atome d'hydrogène et l'autre est choisi parmi le groupe constitué d'un atome d'hydrogène, d'un groupe cyano, d'un groupe cyano-C₁₋₃-alkyle, d'un groupe acétoxy, d'un groupe COOH et d'un groupe COO⁻M⁺, dans lequel M⁺ est un groupe Na⁺, un groupe K⁺ ou un groupe NH4⁺, et
R¹⁶ est choisi parmi le groupe constitué d'un atome d'hydrogène, d'un groupe cyano, d'un groupe alkyle en C₁₋₃, d'un groupe cyano-C₁₋₃-alkyle, d'un groupe acétoxy, d'un groupe COOH et d'un groupe COO⁻M⁺, dans lequel M⁺ est un groupe Na⁺, un groupe K⁺ ou un groupe NH₄⁺, et
COOR est un groupe COOH ou un groupe COO⁻M⁺, dans lequel M⁺ est un groupe Na⁺, un groupe K⁺ ou un groupe NH₄⁺,
comprenant les trois étapes suivantes
(i) convertir un composé de formule 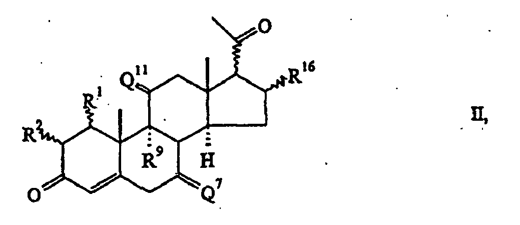 dans laquelle R¹, R², Q⁷, R⁹, Q¹¹ et R¹⁶ sont tels que définis ci-dessus, par une réaction haloforme, éventuellement en présence d'un additif organique polaire, en un composé de formule 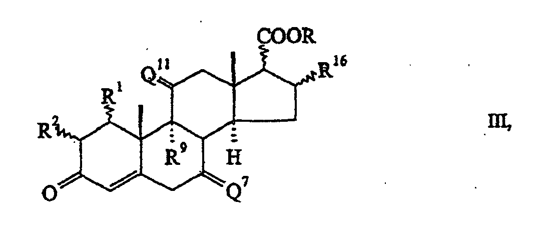 dans laquelle R¹, R², Q⁷, R⁹, Q¹¹, R¹⁶ et COOR sont tels que définis ci-dessus, qui est
(ii) mis à réagir par ozonolyse et un mode opératoire de traitement oxydatif pour donner lieu à un composé de formule 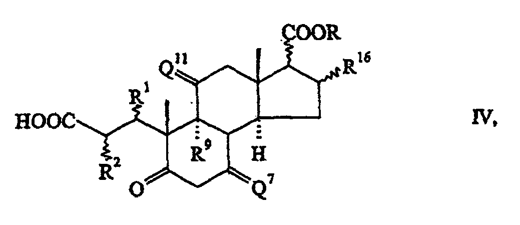 dans laquelle R¹, R², Q⁷, R⁹, Q¹¹, R¹⁶ et COOR sont tels que définis ci-dessus, qui est
(iii) cyclisé avec un composé azoté de formule H₂NR⁴, dans laquelle R⁴ est tel que défini ci-dessus, pour donner lieu à un composé de formule I, dans laquelle R¹, R², R⁴, Q⁷, R⁹, Q¹¹, R¹⁶ et COOR sont tels que définis ci-dessus.

2. Procédé selon la revendication 1, dans lequel la réaction haloforme est une réaction bromoforme.

3. Procédé selon la revendication 1 ou 2, dans lequel l'additif organique polaire est choisi parmi le groupe constitué de l'alcool tert-butylique, de l'acétonitrile et du propionitrile.

4. Procédé selon l'une quelconque des revendications 1 à 3, dans lequel l'étape (ii) est réalisée dans un solvant choisi parmi le groupe constitué du H₂O, d'un alcool en C₁₋₄, de l'acétonitrile, du propionitrile, du chlorure de méthylène et du chloroforme.

5. Procédé selon l'une quelconque des revendications 1 à 4, dans lequel à la fin de l'étape (ii) après le mode opératoire de traitement oxydatif, un réducteur est ajouté au mélange réactionnel en vue d'éliminer les peroxydes.

6. Procédé selon l'une quelconque des revendications 1 à 5, dans lequel le composé azoté de formule H₂NR⁴ dans l'étape (iii) est choisi parmi le groupe constitué de C₁₋₆-alkylamines, de leurs sels solubles, du NH₃, du NH₄Cl, du NH₄Br et du NH₄OAc.

7. Procédé selon l'une quelconque des revendications 1 à 6, dans lequel l'étape (iii) est réalisée à une température de 80 à 150°C, de préférence de 110 à 140°C.

8. Procédé selon les revendications 1 à 7, dans lequel l'étape (iii) est réalisée en présence d'un acide protonique non oxydant choisi parmi le groupe constitué de l'acide acétique, de l'acide propionique, de l'acide butyrique, de l'acide isobutyrique, de l'acide benzoïque, du HCl, du HBr et du HI.

9. Procédé selon les revendications 1 à 8 dans lequel l'étape (iii) est réalisée en présence d'un additif organique polaire choisi parmi le groupe constitué de l'éther tert-butyl-méthylique, d'alcools alkyliques en C₁₋₄, de l'éthylèneglycol, de l'acétonitrile et du diméthylsulfoxyde.

10. Procédé selon l'une quelconque des revendications 1 à 9, pour la préparation de l'acide 3-oxo-4-aza-androst-5-ène-17β-carboxylique de formule I dans laquelle R¹ = R² = R⁴ = R⁷⁻¹ =R⁷⁻² = R⁹ = R¹¹⁻¹ = R¹¹⁻² = R¹⁶ = un atome d'hydrogène et COOR = un groupe COOH (Ia), dans lequel l'étape (ii) est une réaction bromoforme, et dans lequel l'étape (iii) est réalisée par la réaction de l'acide 17β-carboxy-5-oxo-A-nor-3,5-séco-androstan-3-oïque de formule IVa ou d'un sel de celui-ci avec du NH₃ ou un sel d'ammonium soluble, de préférence avec du NH₃ dans de l'éthylèneglycol ou du NH₄OAc dans de l'AcOH, pour donner lieu à un composé Ia ou un sel de celui-ci.
